# EUROPEAN PATENT APPLICATION

(11) **EP 0 829 263 A1**
(43) Date of publication of application: **18.03.1998**
(21) Application number: 97307047.7
(22) Date of filing: 11.09.1997
(51) Int. Cl.: A61K 31/565

(54) **Use of 8,9-dehydroestrone in estrogen therapy**

(30) Priority: 16.09.1996 US 728676
(71) Applicant: AMERICAN HOME PRODUCTS CORPORATION, Madison, New Jersey 07940-0874 (US)
(72) Inventor: Negro-Vilar, Andres, La Jolla, California, 92037 (US); Gast, Jay Michael, Phoenixville, Pennsylvania 19460 (US)
(74) Representative: Connelly, Michael John

(57) **Abstract**

This invention provides a method of providing estrogen therapy or supplementation in a mammal in need thereof, wherein an elevation in prolactin levels concomitant with said estrogenic supplementation is undesirable in said mammal, which comprises administering an effective amount of 8,9-dehydroestrone or a pharmaceutically acceptable salt of its 3-sulfate ester to said mammal.

## Description

### BACKGROUND OF THE INVENTION

The use of naturally occurring estrogenic compositions of substantial purity and low toxicity such as PREMARIN (conjugated equine estrogens) has become a preferred medical treatment for alleviating the symptoms of menopausal syndrome, osteoporosis/osteopenia in estrogen deficient women and in other hormone related disorders. The estrogenic components of the naturally occurring estrogenic compositions have been generally identified as sulfate esters of estrone, equilin, equilenin, 17-β-estradiol, dihydroequilenin and 17-β-dihydroequilenin (U.S. Patent 2,834,712).

Concomitant with the administration of estrogen elevated levels of prolactin may be observed. Prolactin is primarily responsible for milk production during lactation, but also has a wide variety of actions in humans. Excess secretion of this hormone leads to amenorrhea and galactorrhea in women and galactorrhea in men treated with estrogens. Additionally, prolactin elevation has been associated with difficulties in conceiving pregnancies and abnormalities of menstrual function.

8,9-Dehydroestrone is a known compound useful as an intermediate in the synthetic production of estrone by isomerization to 9,11 unsaturation (U.S. Patent 3,394,153) and as an intermediate in the production of 3-cyclopentyloxy-17-ethynyl derivatives of the hormone (U.S. Patent 3,649,621). In addition, 8,9-dehydroestrone is known to possess estrogenic activity and to lower blood lipid levels (U.S. Patent 3,391,169). The alkali metal salts of 8,9-dehydroestrone, 8,9-dehydroestrone-3-sulfate ester and its alkali metal salts, and their use in estrogen replacement therapy, atherosclerosis, and senile osteoporosis are disclosed in U.S. Patents 5,210,081 and 5,288,717.

### DESCRIPTION OF THE INVENTION

This invention provides a method of providing estrogen therapy or supplementation in a mammal in need thereof, wherein an elevation in prolactin levels concomitant with said estrogenic supplementation is undesirable in said mammal, which comprises administering an effective amount of 8,9-dehydroestrone or a pharmaceutically acceptable salt of its 3-sulfate ester to said mammal.

More particularly, the administration of 8,9-dehydroestrone or a pharmaceutically acceptable salt of its 3-sulfate ester is useful in infertility therapies where a rise in prolactin levels, that is seen when estrogenic substances are administered, is undesirable as prolactin elevation makes conception more difficult. For example, 8,9-dehydroestrone or a pharmaceutically acceptable salt of its 3-sulfate ester can be used to prime the endometrium during the pre-implantation period in women undergoing embryo donation during in vitro fertilization and embryo transfer. Such women, who would generally have no ovarian function or artificially suppressed ovarian function, typically undergo several weeks of estrogen therapy during these artificial cycles. In such circumstances, 8,9-dehydroestrone or a pharmaceutically acceptable salt of its 3-sulfate ester would be administered from cycle day one through the end of the cycle in order to cause a proliferative endometrial change, and then to maintain the luteal change after the addition of progesterone and the oocyte transfer which occurs at approximately the mid-cycle stage.

A second use for 8,9-dehydroestrone or a pharmaceutically acceptable salt of its 3-sulfate ester in infertility treatment is its use in the treatment of cervical mucus hostility. In this use, cervical mucus is made more favorable to penetration of sperm by the beneficial effects of estrogen treatment. Estrogen creates a thinner cervical mucus with a slightly higher pH. Estrogenic therapy also increases the size of the cervical mucus matrix allowing sperm penetration to occur. Elevated prolactin levels associated with typical estrogenic administration, however, may create collateral menstrual and other changes, and in that fashion may negate the beneficial effects of the estrogenic administration. As the administration of 8,9-dehydroestrone or a pharmaceutically acceptable salt of its 3-sulfate ester does not cause an increase in prolactin levels, such treatment would not suffer from the deleterious effects of the concomitant rise in prolactin levels. When administered for such use, 8,9-dehydroestrone or a pharmaceutically acceptable salt of its 3-sulfate ester would typically be administered from several days before, until approximately the time that ovulation will occur in normally ovulating individuals.

The administration of 8,9-dehydroestrone or a pharmaceutically acceptable salt of its 3-sulfate ester is also useful in treating the amenorrhea, other ovulatory disorders, and osteopenia that often accompany hyperprolactinemia. The hyperprolacinemia may be caused by the presence of pituitary microprolactinomas or may be idiopathic in nature. In amenorrheic hyperprolactinemic women, mean estradiol levels are often comparable to estradiol levels seen in menopausal women causing such women to be in an absolute or relative estrogen deficiency state. In other women, the presence of early follicular phase levels of estradiol in combination with elevated levels of prolactin is associated with amenorrhea. These women lack the rise in serum estradiol levels typically seen in the mid-follicular, ovulatory, and luteal phase of the cycle. Progressive osteopenia often also develops in these women as a result of their estrogen deficiency. [Klibanski, A., MGH Neuroendocrine Clinical Center Bulletin, Issue 2 (1994); obtained from http://neurosurgery.mgh.harvard.edu/e-f-941.htm]. Elevations of serum prolactin have also been suggested as contributing to osteopenia in young hyperprolactinemic women.

In addition, 8,9-dehydroestrone or a pharmaceutically acceptable salt of its 3-sulfate ester is useful in patients having or susceptible to hyperprolactinemia or having galactorrhea as an estrogen in oral contraceptives, for hormone replacement therapy, in the treatment of tall adolescent girls, in male-to-female transsexuals, and in other disease states or conditions in which estrogenic administration would be beneficial, but an elevation of prolactin levels would be undesirable.

8,9-Dehydroestrone or a pharmaceutically acceptable salt of its 3-sulfate ester is also useful in treating males in need of estrogenic therapy without inducing galactorrhea.

Pharmaceutically acceptable salts of 8,9-dehydroestrone 3-sulfate ester include, but are not limited to, the alkali metal salts, alkaline earth metal salts, ammonium salts, alkylamine salts containing 1-6 carbon atoms or dialkylamine salts containing 1-6 carbon atoms in each alkyl group.

The estrogenicity of 8,9-dehydroestrone-3-sulfate ester sodium salt was disclosed in U.S. Patents 5,210,081 and 5,288,717, which are hereby incorporated by reference.

The neutral effect of 8,9-dehydroestrone and pharmaceutically acceptable salts of its 3-sulfate ester on prolactin levels were established in an in vivo standard pharmaceutical test procedure described below. Ovariectomized (OVX) female Sprague-Dawley rats weighing 150-200g were maintained on a 12h:12h light:dark cycle with food and water available ad libitum. Animals were treated as follows 2-3 weeks following ovariectomy. Animals were divided into treatment groups and received either 8,9-dehydroestrone-3-sulfate ester sodium salt (20 or 100 µg in distilled water), estrone sulfate sodium salt (20 or 100 µg in distilled water), or distilled water vehicle (control group) subcutaneously once daily for three days, and then once again 8 hours before sacrifice. Animals were euthanized on the fourth day and prolactin levels measured by radioimmunoassay.

The following table summarizes the results that were obtained.

| EFFECT ON PROLACTIN LEVELS | |
|---|---|
| Group | Prolactin Levels (ng/ml) |
| Control | 14.29 ± 3.20 |
| Estrone sulfate - 20 µg | 48.47 ± 7.04 |
| Estrone sulfate - 100 µg | 244.87 ± 70.01 |
| 8,9-Dehydroestrone sulfate - 20 µg | 9.47 ± 1.96 |
| 8,9-Dehydroestrone sulfate - 100 µg | 6.77 ± 2.97 |

These results demonstrate that estrone sulfate, a typical estrogen, significantly raised prolactin levels 3-fold and 17-fold when administered at 20 µg and 100 µg, respectively, whereas 8,9-dehydroestrone-3-sulfate ester sodium salt produced no elevation of prolactin levels at either dosage level, demonstrating that 8,9-dehydroestrone, unlike other estrogens, has a neutral effect on prolactin levels.

Based on these results, 8,9-dehydroestrone or a pharmaceutically acceptable salt of its 3-sulfate ester would be useful for providing estrogen therapy or supplementation where an elevation in prolactin levels concomitant with said estrogenic supplementation is undesirable.

8,9-Dehydroestrone or a pharmaceutically acceptable salt of its 3-sulfate ester may be formulated neat for administration. Alternatively a pharmaceutical composition comprising 8,9-dehydroestrone or a pharmaceutically acceptable salt of its 3-sulfate ester in association or combination with a pharmaceutically acceptable carrier may be used. The proportion of the pharmaceutical carrier may be determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. 8,9-Dehydroestrone or a pharmaceutically acceptable salt of its 3-sulfate ester can be administered orally either in liquid or solid composition form. 8,9-Dehydroestrone or a pharmaceutically acceptable salt of its 3-sulfate ester can also be administered as a subdermal implant.

8,9-Dehydroestrone or a pharmaceutically acceptable salt of its 3-sulfate ester may be administered rectally in the form of a conventional suppository. For administration by intranasal or intrabronchial inhalation or insufflation, the antioxidants of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

In addition, 8,9-dehydroestrone or a pharmaceutically acceptable salt of its 3-sulfate ester may be employed as a solution, cream, or lotion by formulation with pharmaceutically acceptable vehicles containing 0.1 - 5 percent, preferably 2%, of active compound which may be administered to a fungally affected area.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg - 500 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

## Claims

1. Use of 8,9-dehydroestrone or a pharmaceutically acceptable salt of its 3-sulfate ester to make a medicament for providing estrogen therapy or supplementation in a mammal in need thereof, wherein an elevation in prolactin levels concomitant with said estrogen therapy or supplementation is undesirable in said mammal.

2. Use of 8,9-dehydroestrone or a pharmaceutically acceptable salt of its 3-sulfate ester to make a medicament for providing estrogen therapy in the treatment of infertility in a mammal in need thereof.

3. Use of 8,9-dehydroestrone or a pharmaceutically acceptable salt of its 3-sulfate ester to make a medicament for treating ovulatory disorders in a mammal having hyperprolactinemia.

4. Use according to claim 3, wherein the ovulatory disorder is amenorrhea.

5. Use of 8,9-dehydroestrone or a pharmaceutically acceptable salt of its 3-sulfate ester to make a medicament for treating osteopenia in a mammal having hyperprolactinemia.

6. Use of 8,9-dehydroestrone or a pharmaceutically acceptable salt of its 3-sulfate ester to make a medicament for providing estrogen therapy or supplementation in a mammal susceptible to developing hyperprolactinemia.

7. Use of 8,9-dehydroestrone or a pharmaceutically acceptable salt of its 3-sulfate ester to make a medicament for providing estrogen therapy or supplementation in a mammal having galactorrhea.
